# NEUE EUROPÄISCHE PATENTSCHRIFT

(11) **EP 0 804 933 B2**
(45) Veröffentlichungstag und Bekanntmachung des Hinweises auf die Entscheidung über den Einspruch: **11.03.2009**
(45) Hinweis auf die Patenterteilung: 07.04.2004
(21) Anmeldenummer: 97106568.5
(22) Anmeldetag: 21.04.1997
(51) Int. Cl.: A61L 26/00, A61K 38/36

(54) **Lagerstabile Fibrinogen-Präparate**
Storage-stable fibrinogen preparations
Préparations de fibrinogène stables au stockage

(30) Priorität: 30.04.1996 DE 19617369
(43) Veröffentlichungstag der Anmeldung: 05.11.1997
(73) Patentinhaber: Baxter Aktiengesellschaft, 1221 Wien (AT)
(72) Erfinder: Seelich, Thomas, Dr., 1030 Wien (AT)
(74) Vertreter: Perrey, Ralf

(56) Entgegenhaltungen:
- EP-A- 0 085 923
- EP-A- 0 345 246
- US-A- 4 377 572
- US-A- 4 909 251
- US-A- 5 290 918
- REDL H. ET AL: 'Vergleich zweier Fibrinkleber' DIE MEDIZINISCHE WELT Bd. 36, 1985, Seiten 769 - 776
- MOMMAERTS W.F.H.: 'ON THE NATURE OF FORCES OPERATING IN BLOOD CLOTTING' THE JOURNAL OF GENERAL PHYSIOLOGY Bd. 29, 1946, Seiten 113 - 122

## Beschreibung

Die Erfindung betrifft lagerstabile Fibrinogenpräparate zur Bereitung von konzentrierten Fibrinogenlösungen zur Verwendung als Gewebeklebstoff oder zur Bereitung von Fibrinogenlösungen für andere Anwendungen, beispielsweise für Infusionszwecke.

Die lagerstabilen Fibrinogenpräparate können entweder in lyophilisierter Form oder als tiefgefrorene (insbesondere hochkonzentrierte) Fibrinogenlösungen vorliegen, und haben den Vorteil, dass sie einfacher und rascher als bisher bekannte vergleichbare Präparate zu gebrauchsfertigen Fibrinogen- bzw. Gewebeklebstofflösungen rekonstituiert bzw. verflüssigt werden können.

Die Erfindung beinhaltet weiters auch die aus den erfindungsgemässen Präparaten erhältlichen Fibrinogen- bzw. Gewebeklebstofflösungen.

Gewebeklebstoffe auf Basis von Fibrinogen werden zum nahtlosen bzw. nahtunterstützenden Verbinden von menschlichen oder tierischen Gewebe- oder Organteilen, zur Wundversiegelung, Blutstillung und Förderung der Wundheilung eingesetzt.

Ihre Wirkungsweise beruht darauf, dass durch Einwirkung von Thrombin das im gebrauchsfertigen, flüssigen Gewebeklebstoff enthaltene (lösliche) Fibrinogen in (unlösliches) Fibrin übergeführt und der ebenfalls enthaltene Faktor XIII zu Faktor XIIIa aktiviert wird. Dieser vernetzt das gebildete Fibrin zu einem Hochpolymer, was für die Wirksamkeit des Gewebeklebstoffes wesentlich ist. Die benötigte Thrombinaktivität kann entweder aus dem zu klebenden Gewebe (den Wundflächen) selbst stammen oder in Form einer Thrombin und Ca²⁺-Ionen enthaltenden Lösung dem Gewebeklebstoff bei der Klebung zugesetzt werden.

Gewebeklebstoffe auf Basis von Fibrinogen sind bereits aus AT-B-359 653, AT-B-359 652 und AT-B-369 990 bekannt. Sie enthalten neben Fibrinogen und Faktor XIII noch weitere Proteine, wie Fibronectin und Albumin und gegebenenfalls Antibiotika. Gewebeklebstoffe kommen entweder in Form von tiefgefrorenen Lösungen oder als Lyophilisat in den Handel, da sie als flüssige Lösungen wenig stabil und nicht für längere Zeit haltbar sind. Dieser Umstand führt dazu, dass die Handelsprodukte vor ihrer Applikation entweder aufgetaut, d.h. verflüssigt, oder aus ihrem Lyophilisat rekonstituiert werden müssen. Beide Massnahmen sind mit einem nicht unerheblichen Zeitaufwand verbunden.

Für die optimale Klebung ist ein Gehalt an Fibrinogen von mindestens 70 mg/ml der gebrauchsfertigen Gewebeklebstofflösung und ein Gehalt an Faktor XIII erforderlich, wobei das Verhältnis des Faktors XIII zu Fibrinogen, ausgedrückt in Einheiten des Faktors XIII pro Gramm Fibrinogen, mindestens 80 beträgt.

Solche Präparate ermöglichen u.a. eine sichere Blutstillung, eine gute Haftfähigkeit des Klebstoffes an den Wund- bzw. Gewebsflächen, eine hohe Belastbarkeit der Klebungen bzw. Wundversiegelungen, eine vollkommene Resorbierbarkeit des Klebstoffes im Verlauf des Wundheilungsprozesses, und sie haben wundheilungsfördernde Eigenschaften.

Der erforderliche hohe Fibrinogengehalt bewirkt allerdings, dass die lyophilisierten bzw. flüssig-tiefgefrorenen Fibrinogenpräparate erst allmählich und bei erhöhter Temperatur - im allgemeinen erst über 25°C, zumeist über 30°C - zum gebrauchsfertigen, flüssigen Gewebeklebstoff rekonstituiert bzw. verflüssigt ("aufgeschmolzen") werden können.

Ärztlicherseits besteht der Wunsch nach einer raschen Verfügbarkeit der gebrauchsfertigen Gewebeklebstofflösung, da dies insbesondere in chirurgischen Notsituationen von entscheidender Bedeutung sein kann.

Für die Bereitstellung der gebrauchsfertigen Lösung sollen ausserdem möglichst wenig Handgriffe erforderlich sein, um bei Operationen das Hilfspersonal möglichst wenig zu belasten, und es wird als Vorteil empfunden, wenn dazu keine zusätzlichen Hilfsmittel erforderlich sind.

Weiters besteht ärztlicherseits der Wunsch nach gebrauchsfertigen Gewebeklebstofflösungen, die trotz ihres hohen Fibrinogengehaltes bereits bei Raumtemperatur, etwa bei 20°C, dünnflüssig genug sind, um leicht verarbeitet werden zu können.

Relativ dünnflüssige Gewebeklebstofflösungen sind insbesondere dann von Vorteil, wenn der Gewebeklebstoff durch dünne Katheter in das Innere von Körperhöhlen eingeführt und dort appliziert werden soll, oder auch bei Anwendung der Sprühtechnik, wobei der Gewebeklebstoff versprüht und als dünne Schicht auf Wundflächen aufgebracht wird. Entsprechende Applikationsgeräte für Gewebeklebstoffe stehen bereits zur Verfügung (EP 0 315 222, EP 0 455 626).

Die Löslichkeit der Fibrinogenzubereitungen kann sich durch die Anwendung von Virusinaktivierungsverfahren noch verschlechtern. Diese werden vorzugsweise derart durchgeführt, dass das lyophilisierte Material einer Hitzebehandlung, beispielsweise gemäss EP 0 159 311, unterzogen wird.

Es hat daher nicht an Versuchen gefehlt, die Auflösbarkeit von lyophilisierten Fibrinogenzubereitungen zu verbessern.

Es ist bekannt, dass die Auflösbarkeit von Lyophilisaten durch bestimmte Zusätze verbessert werden kann. So beschreibt EP-A-0 345 246 eine lyophilisierte Fibrinogenzubereitung, die neben Fibrinogen noch mindestens ein biologisch verträgliches Tensid enthält. Der Zusatz von Tensiden bewirkt eine verbesserte Benetzung des Lyophilisates mit dem Lösungsmittel, wodurch zwar die Lösungsgeschwindigkeit, nicht aber die Löslichkeit des Fibrinogens an sich - bei einer gegebenen Temperatur - verbessert wird. Solche Präparate müssen daher ebenfalls bei Temperaturen über 25°C, vorzugsweise bei 37°C, rekonstituiert werden.

EP-A-085 923 beschreibt eine lyophilisierte Fibrinogenzubereitung, die neben Fibrinogen noch eine Substanz enthält, die einen Harnstoff- oder einen Guanidinrest aufweist. Es hat sich allerdings gezeigt, dass nach diesem Vorschlag hergestellte lyophilisierte Gewebeklebstoffpräparationen zytotoxisch wirken, das Wachstum von Fibroblasten hemmen und zu einer veränderten, unphysiologischen Fibrinstruktur führen, wodurch die erwünschte Elastizität des Fibrins verlorengeht (vgl. Redl et al, Medizinische Welt 36, 769-76 (1985). Mit der Wachstumshemmung der Fibroblasten, also derjenigen Zellen, die den Wundheilungsprozess einleiten, gehen die erwünschten wundheilungsfördernden Eigenschaften von Gewebeklebstoffen auf Fibrinogenbasis verloren. Durch die fehlende Elastizität des entstandenen Fibrins wird weiters die geforderte hohe Belastbarkeit der Klebungen in vivo in Frage gestellt.

Die vorliegende Erfindung stellt sich die Aufgabe, lagerstabile Fibrinogenpräparate in lyophilisierter oder flüssig-tiefgefrorener Form zur Verfügung zu stellen, die rasch und in einfacher Weise - vorzugsweise ohne Verwendung von zusätzlichen Hilfsmitteln wie Wärme- und/oder Rührgeräten - zu gebrauchsfertigen Fibrinogen- bzw. Gewebeklebstofflösungen rekonstituiert bzw. verflüssigt werden können, wobei die gebrauchsfertigen Gewebeklebstofflösungen (mit einem Fibrinogengehalt von mindestens 70 mg/ml) bereits unter 25°C dünnflüssig genug sind, um leicht verarbeitet werden zu können, und trotzdem nicht die oben erörterten Nachteile bekannter Präparate, insbesondere solcher entsprechend EP-A-085 923, aufweisen.

Die gestellte Aufgabe wird erfindungsgemäss dadurch gelöst, dass die Präparationen neben Fibrinogen und gegebenenfalls weiteren Proteinen sowie Hilfs- und Zusatzstoffen mindestens eine Substanz enthalten, die die Löslichkeit von Fibrinogen verbessert bzw. deren Verflüssigungstemperatur erniedrigt und die Viskosität einer gebrauchsfertigen Gewebeklebstofflösung bei Raumtemperatur absenkt. Dabei erfolgt die Auswahl der Substanz nicht nur.nach deren Wirkung auf die Löslichkeit von Fibrinogen, sondern auch nach der Bildung einer physiologischen Fibrinstruktur, wenn das gelöste Präparat mit einem Aktivator reagiert und Fibrinclots gebildet werden. Der diesbezügliche Nachweis kann nach Mischen der Fibrinogenlösung mit dem gleichen Volumen einer Thrombin-CaCl₂-Lösung (im wesentlichen bestehend aus 4 I.E. Thrombin und 20 bis 40 µmol CaCl₂ pro ml) erbracht werden, wenn die gebildeten Clots eine physiologische Fibrinstruktur, also die typische, räumlich verzweigte Struktur aufweist, wie sie bei Einwirkung von Thrombin auf Fibrinogen unter physiologischen Bedingungen, d.h. bei einer Ionenstärke von etwa 0,15 und einem pH-Wert von etwa 7,4 gebildet wird. Makroskopisch ist die physiologische Struktur durch einen undurchsichtigen und zähelastischen Clot gekennzeichnet. REM-Aufnahmen von typischen physiologischen bzw. nicht physiologischen Fibrinclots werden beispielsweise in der Publikation von Redl et al. Medizinische Welt 36, 769-76 (1985) gezeigt.

Das erfindungsgemässe Präparat ist derart zusammengesetzt, dass es bei Anwendung als Gewebeklebstoff keine zytotoxische Wirkung hat, also sehr gut zellverträglich ist, ein gutes Zellwachstum ermöglicht und damit eine ideale Voraussetzung für eine gute Wundheilung bietet. Der Nachweis dafür kann nach dem Verdünnen des Gewebeklebstoffes mit dem gleichen Volumen einer halbisotonen oder isotonen Natriumchloridlösung erbracht werden, wenn keine schädigende Wirkung auf Fibroblasten nachweisbar ist.

Eine Fibrinogen- bzw. Gewebeklebstofflösung wird im allgemeinen als nicht-zytotoxisch bezeichnet, wenn sie im Zellüberschichtungstest nach Redl et al. (siehe oben) keine schädigende Wirkung auf Fibroblasten ausübt.

Weiters ist das erfindungsgemässe Präparat vorzugsweise so zusammengesetzt, dass auch alle weiteren Anforderungen, die an einen Gewebeklebstoff zu stellen sind, nämlich
- Virussicherheit
- hohe Belastbarkeit der Klebungen bzw. Wundversiegelungen, sowie sichere und anhaltende Blutstillung,
- regelbare Haltbarkeit der Klebungen im Körper, durch einen variablen Gehalt an Plasminogen bzw. Fibrinolyseinhibitor,
- vollkommene Resorbierbarkeit des Klebstoffes im Verlaufe des Wundheilungsprozesses,
- wundheilungsfördernde Eigenschaften,

erfüllt sind, d.h. dass durch den erfindungsgemässen Gehalt des Gewebeklebstoffes an einer Substanz, die die Löslichkeit der Präparation verbessert bzw. deren Verflüssigungstemperatur erniedrigt und die Viskosität der gebrauchsfertigen Gewebeklebstofflösung bei Raumtemperatur absenkt, die sonstigen erwünschten biochemischen und physikalischen Eigenschaften des Gewebeklebstoffes praktisch nicht beeinträchtigt werden.

Dies kann unter anderem durch folgende weitere Testungen nachgewiesen werden:
- Gerinnungsreaktion nach Mischen mit einer Thrombinlösung (Gerinnungszeit)
- Vernetzung der Fibrin-γ- und α-Ketten
- Fibrinolyseresistenz
- Reissfestigkeit
- Klebefestigkeit.

Unter Verflüssigungstemperatur wird diejenige Temperatur verstanden, bei der beim Erwärmen einer tiefgefrorenen konzentrierten Fibrinogenlösung Verflüssigung eintritt.

Das erfindungsgemässe lagerstabile Fibrinogenpräparat in flüssig-tiefgefrorener Form enthält eine löslichkeitverbessernde Substanz, so dass es bei einer Temperatur von 0 bis 25°C, vorzugsweise unter 20°C, besonders bevorzugt unter 15°C, zu einer Lösung mit einem Fibrinogengehalt von mindestens 70mg/ml verflüssigbar ist. Eine erniedrigte Verflüssigungstemperatur bedeutet gleichzeitig eine raschere Verflüssigung einer tiefgefrorenen, konzentrierten Fibrinogenlösung, wenn diese beispielsweise einer Umgebungstemperatur von 20°C - 25°C (Raumtemperatur) ausgesetzt wird. Dies gilt insbesondere für die erfindungsgemässen tiefgefrorenen Präparate, die in gebrauchsfertigen, sterilen Einmalspritzen abgefüllt und aus Gründen der Sterilität zweifach in Plastikfolien eingeschweisst sind.

Durch die notwendige doppelte Umhüllung ist der Wärmeübergang erschwert, weshalb solcherart abgepackte, tiefgefroren gelagerte Präparate bisher nur mit Hilfe eines Wasserbades (37°C) in akzeptabler Zeit verflüssigt, d.h. gebrauchsfertig gemacht werden konnten. Die Verwendung eines Wasserbades ist jedoch umständlich und mit Nachteilen verbunden, z.B. hinsichtlich der Aufrechterhaltung steriler Bedingungen.

Aber auch die erfindungsgemässen lyophilisierten Fibrinogen- bzw. Gewebeklebstoffpräparationen haben den Vorteil, dass diese bei Raumtemperatur ohne besondere Hilfsmittel (wie z.B. dem kombinierten Wärme- und Rührgerät gemäss AT-B-371 719) in akzeptabler Zeit, d.h. in einem Zeitraum von etwa einer halben Minute bis zu 15 Minuten, vorzugsweise weniger als 7 Minuten, besonders bevorzugt weniger als 5 Minuten zu einer Lösung mit einem Fibrinogengehalt von mindestens 70 mg/ml, rekonstituiert werden können. Bisher war dies nur bei Präparationen gemäss EP-A-085 923 der Fall; jedoch weisen gerade diese Präparationen, wie oben erwähnt, andere schwerwiegende Nachteile auf.

Weiters erlauben die erfindungsgemässen Präparate auch die besonders rasche und einfache Bereitstellung von Fibrinogenlösungen für andere Zwecke, beispielsweise zur Infusion.

Es wurde überraschenderweise gefunden, dass eine Reihe von Substanzen die weiter oben beschriebenen Anforderungen erfüllen, d.h. die Löslichkeit von Fibrinogen erhöhen, die Verflüssigungstemperatur von konzentrierten, tiefgefrorenen Fibrinogen- bzw. Gewebeklebstofflösungen, sowie deren Viskosität bei Raumtemperatur erniedrigen ohne die weiter oben beschriebenen unerwünschten Nebenwirkungen von Zusatzstoffen gemäss EP-A-085 923 hervorzurufen.

Die erfindungsgemässen Präparationen enthalten eine oder mehrere der Substanzen ausgewählt aus der Gruppe der Nukleinbasen, Nukleoside oder Nukleotide und Benzoesäure, p-Aminobenzoesäure (Vitamin H'), p-Aminosalicylsäure, Hydroxybenzoesäure, Hydroxysalicylsäure, Phenylalanin, Prokain, Niacin, Niacinamid, Picolinsäure, Vitamin B6 (Pyridoxin), Hydroxypyridinen, Pyridin-dicarbonsäuren, Pyridinsulfonsäuren, Piperidin-carbonsäureester, Pyrimidin, Barbitursäure, Uracil, Uridin, Uridinphosphaten, Thymin, Cytosin, Cytidin, Hydroxypyrimidinen, Thiamin (Vitamin B1), Morpholin, Pyrrolidon, Imidazol, Hydantoin, Pyrazol-dicarbonsäuren, Phenazon, Adenosin, Adenosin-Phosphaten, Inosin, Guanosin-Phosphaten, Brenzschleimsäure (Furan-2-carbonsäure), Ascorbinsäure (Vitamin C) und Xantosin, vorzugsweise in einer Menge von 0,03 mmol - 3 mmol, am meisten bevorzugt in einer Menge von 0,07 mmol - 1,4 mmol/g Fibrinogen.

Zur Bereitung von Infusionslösungen aus lyophilisierten Fibrinogenpräparaten ist es vorteilhaft entsprechend höhere Verhältnisse der Menge an der Substanz bezogen auf Fibrinogen zu wählen. Beispielsweise kann die Substanz in einer Menge von 0,12-12 mmol, vorzugsweise 0,28-5,6 mmol/g Fibrinogen, enthalten sein.

Die Art und Menge der Substanz ist so zu wählen, dass das gebrauchsfertige Gewebeklebstoffpräparat bei Raumtemperatur dünnflüssig genug ist, um problemlos versprüht werden zu können, entsprechend einer Viskosität von weniger als 400 mm²/s (400 cSt.), vorzugsweise weniger als 300 mm²/s (300 cSt). und nach Mischen der gebrauchsfertigen Gewebeklebstofflösung mit einer Thrombin-CaCl₂-Lösung im Verhältnis 1:1 physiologische Clots gebildet werden.

Die erfindungsgemässen Fibrinogenpräparate zeichnen sich weiters durch ihren relativ geringen Salzgehalt aus, so dass die Osmolarität der konzentrierten Gewebeklebstofflösung vorzugsweise weniger als 500 mOsm, am meisten bevorzugt weniger als 400 mOsm beträgt. Diese Begrenzung ist notwendig, um die erwünschte gute Zellverträglichkeit (Abwesenheit zytotoxischer Eigenschaften) zu ermöglichen. Erst durch die erfindungsgemässen Substanzen wurde es möglich, lyophilisierte Fibrinogenpräparate mit relativ niedrigem Salzgehalt zu schaffen, die, wie weiter oben beschrieben, trotzdem problemlos bei Raumtemperatur zu gebrauchsfertigen, relativ dünnflüssigen Gewebeklebstofflösungen (mit einem Fibrinogengehalt von mindestens 70 mg/ml) rekonstituiert werden können, **und** gut zellverträglich sind.

Bisher bekannt gewordene Präparate sind entweder bei Raumtemperatur gut löslich, jedoch zellschädigend (Beriplast®, Biocol®, Bolheal HG-4®) oder gut zellverträglich, jedoch erst bei erhöhter Temperatur - vorzugsweise bei 37°C - rekonstituierbar (Tissucol®).

Die erfindungsgemässen Präparate können aufgrund ihrer Zusammensetzung auch als virussichere Präparate zur Verfügung gestellt werden, die sich auch trotz Vorbehandlungen zu Inaktivierung und/oder Abreicherung von Viren gut lösen. Besonders wirksame Behandlungsverfahren sind mehrstufige Hitzebehandlungsverfahren, z.B. Dampfbehandlung bei 60°C und 80°C gemäss EP-159 311 oder aber Kombinationen von chemischen und/oder physikalischen Behandlungsverfahren.

Besonders bevorzugt ist ein Hitzebehandlungsverfahren bevor die löslichkeitverbessernde Substanz dem Präparat zugesetzt wird und gegebenenfalls eine Nanofiltration vor dem Abfüllen in die Endbehälter.

Vorzugsweise enthält das erfindungsgemässe Präparat weiterhin Faktor XIII, aber auch Fibronektin und gegebenenfalls geringe Mengen an Plasminogen, was u.a. für den Verlauf der Wundheilung von Vorteil sein kann.

Andererseits kann in bestimmten Fällen auch ein erfindungsgemässes Präparat im wesentlichen nur aus Fibrinogen bestehen, also als einzige Wirksubstanz Fibrinogen enthalten.

Eine weitere bevorzugte Ausführungsform betrifft einen Gewebeklebstoff auf Basis von Fibrinogen und einem Plasminogen-Aktivator-Inhibitor oder Plasmininhibitor, wie Aprotinin, α₂-Plasmininhibitor, α₂-Makroglobulin und dergleichen. Die gebrauchsfertige Gewebeklebstofflösung enthält im allgemeinen 70 bis 120 mg Fibrinogen, gegebenenfalls 0,50 bis 50 E Faktor XIII, gegebenenfalls 0,5 bis 15 mg Fibronectin, 0 bis 150 µg Plasminogen und 0 bis 20.000 KIU Aprotinin, vorzugsweise 1.000 bis 15.000 KIU Aprotinin pro ml. Ein bevorzugtes Präparat enthält weiters geringe Tensidkonzentrationen zur besseren Benetzbarkeit des lyophilisierten Präparates bzw. zur Verbesserung der tiefgefrorenen Präparate.

Die Erfindung wird durch die folgenden Beispiele näher erläutert.

### Beispiel 1: Verflüssigungstemperaturen verschiedener tiefgefrorener Gewebeklebstofflösungen

Ein virusinaktiviertes (dampfbehandeltes) Gewebeklebstoffpräparat wurde nach an sich bekannten Methoden (vgl. AT-B-369 653, EP 0 345 246) folgendermassen hergestellt:

Ein Plasmakryopräzipitat aus gepooltem humanem Citratplasma wurde mit einer Pufferlösung (pH 6,5) enthaltend 6,6 g Natriumcitrat.2H₂O, 3,4 g NaCl, 10 g Glycin, 25.000 KIE Aprotinin und 200 I.E. Heparin pro 1 bei 2°C gewaschen und zentrifugiert. Der Niederschlag wurde mit einer weiteren Pufferlösung enthaltend 9,0 g Glycin, 1,0 g Natriumcitrat.2H₂O, 25.000 KIE Aprotinin und 0,2 g Triton WR 1339, auf eine Proteinkonzentration von 42 g/l eingestellt. Es wurden sodann pro l 4,5 g reines Humanalbumin und 15.000 E Faktor XIII zugesetzt und der pH-Wert auf 7,3 eingestellt. Die Lösung wurde im Ganzen lyophilisiert, auf einen Feuchtigkeitsgehalt von 7,5% eingestellt und unter N₂-Atmospäre 10 Stunden lang auf 60°C erhitzt. Das solcherart virusinaktivierte Material wurde mit destilliertem H₂O zu einer Proteinkonzentration von 47 g/l gelöst, sterilfiltriert, und nochmals lyophilisiert. Portionen des lyophilisierten Materials wurden mit destilliertem H₂O ohne oder mit den in Tabelle 1 angegebenen Zusätzen zu einer Fibrinogenkonzentration von 85 mg/ml gelöst (=gebrauchsfertige Gewebeklebstofflösung), je 2 ml in Röhrchen verfüllt und tiefgefroren.

Die Verflüssigungstemperatur der so erhaltenen tiefgefrorenen Gewebeklebstofflösungen wurde im folgenden Verflüssigungstest bestimmt:

Die tiefgefrorenen Proben werden zunächst in einem auf 10°C temperierten Wasserbad 30 Minuten lang inkubiert. Danach wird die Temperatur in Abständen von 30 Minuten um jeweils 2,5°C erhöht. Nach jeder Inkubationsperiode wird der Aggregatzustand der Proben durch Kippen der Röhrchen beurteilt. Der Übergang vom festen in den flüssigen Zustand erfolgt nicht abrupt, sondern über einen Bereich mehrerer Temperaturstufen, wobei gallertige und zähflüssige Zwischenzustände durchlaufen werden.

Entsprechend diesem Test wird eine Probe erst dann als "flüssig" bezeichnet, wenn sich beim Kippen des Röhrchens praktisch sofort wieder ein waagrechter Flüssigkeitsspiegel einstellt, d.h. wenn die Probe beim Fliessen keinen "Bauch" bildet.

Mit Hilfe dieses einfachen Tests kann beurteilt werden, ob eine Gewebeklebstofflösung bei einer gegebenen Temperatur ausreichend dünnflüssig ist, um problemlos angewendet werden zu können.

Die Ergebnisse sind in Tabelle 1 zusammengestellt.

Zusätzlich wurde überprüft, ob die angeführten Gewebeklebstofflösungen nach Mischen mit dem gleichen Volumen einer Thrombin-CaCl₂-Lösung (4 I.E. Thrombin und 40 µmol CaCl₂ pro ml, hergestellt aus Thrombin 500, Immuno AG) wie erwünscht physiologische, undurchsichtige, zähelastische Clots bilden.

Dies war bei allen angeführten Gewebekebstofflösungen der Fall.

**Tabelle 1:**

| **Ergebnisse:** | | | |
|---|---|---|---|
| **zugesetzte Substanz** | **Gruppe bzw. Verbindung** | **Konz. mM** | **Verflüss.- Temp. °C** |
| kein Zusatz (Vergleich) | - | - | 27,5 |
| Benzoesäure (Na-Salz ) | Benzol- | 200 | 15 |
| p-Aminobenzoesäure (Na-Salz) | Benzol-, Vitamin | 25 | 20 |
| | | 50 | 12,5 |
| p-Aminosalicylsäure (Na-Salz) | Benzol- | 50 | 15 |
| | | 100 | 10 |
| p-Hydroxybenzoesäur e (Na-Salz) | Benzol- | 50 | 15 |
| | | 100 | 10 |
| Phenylalanin | Benzol- | 100 | 20 |
| Procain, HCl | Benzol- | 50 | 15 |
| Niacin (Na-Salz) | Pyridin- | 50 | 22,5 |
| | | 100 | 10 |
| | Pyridin-, Vitamin | 50 | 12,5 |
| Niacinamid | | 100 | 10 |
| | | 200 | <10 |
| Picolinsäure | Pyridin- | 50 | 22,5 |
| | | 100 | 10 - 12,5 |
| Pyridoxin, HCl Adermin, Vitamin B₆ | Pyridin-, Vitamin | 50 | ≤ 10 |
| Pyridin-2,6-dicarbonsäure.Na | Pyridin- | 50 | 10 |
| 2-Hydroxypyridin | Pyridin- | 50 | 15 |
| | | 100 | 12,5 |
| 3-Hydroxypyridin | Pyridin- | 200 | 17,5 |
| 4-Hydroxypyridin | Pyridin- | 50 | 10 |
| Pyridin-2,3-dicarbon-säure.Na | Pyridin- | 50 | 17,5 - 20 |
| Pyridin-3-sulfonsäure.Na | Pyridin- | 50 | 20 |
| | | 100 | 15 |
| | | 200 | 10 |
| Pyperidin-4 carbon-säureethylester | Piperidin | 100 | 20 |
| Pyrimidin | Pyrimidin- | 50 | 22,5 |
| | | 100 | 12,5 |
| Barbitursäure.Na | Pyrimidin- | 50 | 10 |
| Uracil | Pyrimidin-, Nucleinbase | 25 | 22,5 |
| Uridin | Pyrimidin-Nucleosid | 50 | 10 |
| Uridin-5'-phosphat | Pyrimidin- | 25 | 10 |
| Thymin | Pyrimidin-, Nucleinbase | 25 | 22,5 |
| Cytosin | Pyrimidin-, Nucleinbase | 25 | 10 |
| Cytidin | Pyrimidin-, Nucelotid | 50 | 12,5 |
| 4-Hydroxypyrimidin (4,3H-Pyrimidon) | Pyrimidin- | 50 | 10 |
| Morpholin | Morpholin- | 100 | 15 |
| α-Pyrrolidon | Pyrrol- | 50 | 10-12,5 |
| | | 100 | 10 |
| Imidazol | Imidazol- | 100 | 17,5 |
| | | 200 | 12,5 |
| Hydantoin | Imidazol- | 50 | 22,5 |
| | | 100 | 10 |
| Pyrazol-3,5-dicarbonsäure | Pyrazol- | 25 | 12,5 |
| Phenazon, Antipyrin | Pyrazol- | 50 | 12,5 |
| Adenosin | Purin-, Nucelosid | 25 | 12,5 |
| Inosin | Purin-, Nucleosid | 25 | 12,5 |
| | | 50 | 10 |
| Adenosin-5'-phosphat | Purin-, Nucleotid | 6,25 | 22,5 |
| | | 12,5 | 10 |
| Guanosin-5'-phosphat | Purin-, Nucleotid | 6,25 | 15 |
| | | 12,5 | 10 |
| Furan-2-carbonsäure, Brenzschleimsäure | Furan- | 100 | 17,5 |
| | | 200 | 10 |
| Furan-3-carbonsäure | Furan- | 100 | 22,5 |
| | | 200 | 12,5 |
| Ascorbinsäure | Furan-, Vitamin | 50 | 15 |
| Xanthosin | Purin-, Nucleosid | 25 | 12,5 |
| | | 50 | 10 |

### Beispiel 2: Einfluss von Niacinamid auf die Viskosität von Gewebeklebstofflösungen

Analog zu Beispiel 1 wurden flüssig-tiefgefrorene Gewebeklebstofflösungen (Gehalt an gerinnbarem Protein: 81mg/ml) mit unterschiedlichem Gehalt an Niacinamid hergestellt.

Nach Auftauen wurde die kinematische Viskosität bei verschiedenen Temperaturen in einem Kapillarviskosimeter gemessen.

Die Ergebnisse sind in Tabelle 2 zusammengestellt.

**Tabelle 2**

| Abhängigkeit der kinematischen Viskosität einer Gewebeklebstofflösung vom Gehalt an Niacinamid bei verschiedenen Temperaturen: | | | | | | | | | |
|---|---|---|---|---|---|---|---|---|---|
| **Niacinamid (mM)** | **Viskosität mm²/s (cSt) bei °C** | | | | | | | | |
| | **10,0** | **12,5** | **15,0** | **17,5** | **20,0** | **22,5** | **25,0** | **30,0** | **37,0** |
| 0(Vergleich) | | | | | 812 | 432 | 238 | 107 | 58,6 |
| 25 | | | | 1152 | 531 | 294 | 184 | 92,6 | 54,8 |
| 50 | | | 850 | 471 | 277 | 184 | 132 | 81,3 | 52,9 |
| 100 | | 1035 | 556 | 332 | 216 | 152 | 114 | 73,7 | 52,0 |
| 200 | 646 | 424 | 286 | 207 | 152 | 118 | 93,5 | 66,2 | |

### Beispiel 3: Einfluss von Pyridoxin.HCl auf die Viskosität von Gewebeklebstofflösungen

Durchführung analog Beispiel 2; Ergebnisse Tabelle 3.

**Tabelle 3:**

| Abhängigkeit der kinematischen Viskosität einer Gewebeklebstofflösung vom Gehalt an Pyridoxin.HCl. | | | | | | | | |
|---|---|---|---|---|---|---|---|---|
| **Pyridoxin. HCl (mM)** | **Viskosität mm²/s (cSt) bei °C** | | | | | | | |
| | **12,5** | **15** | **17,5** | **20** | **22,5** | **25** | **30** | **37** |
| 0(Vergleich) | | | | 812 | 432 | 238 | 107 | 59 |
| 12,5 | | | | 695 | 372 | 214 | 97 | 57 |
| 25 | | | 945 | 459 | 252 | 159 | 85 | 51 |
| 50 | | 1151 | 583 | 323 | 196 | 147 | 85 | |
| 100 | 803 | 499 | 315 | 215 | 153 | 116 | 63 | 47 |

### Beispiel 4: Einfluss von Niacinamid auf die Rekonstitutionszeit von lyophilisierten Gewebeklebstoffpräparationen

Ein lyophilisiertes, virusinaktiviertes Gewebeklebstoffpräparat wurde bis zum Erhitzungsschritt analog Beispiel 1 hergestellt.

Das Material wurde danach mit destilliertem H₂O bzw. wässrigen Niacinamidlösungen unterschiedlicher Konzentration zu einer Proteinkonzentration von 30 g/l gelöst, sterilfiltriert, in Portionen zu je 4,0 ml in sterile Endbehälter (Glasfläschchen) abgefüllt und lyophilisiert.

Nach Rekonstitution mit 1,0 ml H₂O oder wässriger Aprotininlösung wurden gebrauchsfertige Gewebeklebstofflösungen mit einem Fibrinogengehalt von 85 mg/ml erhalten. Die benötigten Rekonstitutionszeiten bei Raumtemperatur, unter leichtem Schütteln von Hand, wurden an mehreren Fläschchen jeder Variante bestimmt. Die Ergebnisse sind in Tabelle 4 zusammengestellt.

Aprotinin bis zu einer Konzentration von 3000 KIE/ml im Lösungsmittel hatte auf die Rekonstitutionszeit keinen merklichen Einfluss.

**Tabelle 4**

| Rekonstitutionszeiten von lyophilisierten Gewebeklebstoffpräparationen in Abhängigkeit vom Gehalt an Niacinamid. | |
|---|---|
| **Niacinamid, Endkonzentrat (mM)** | **Rekonstitutionszeit bei RT (Min.)** |
| 0 (Vergleich) | >20 |
| 50 | 9-11 |
| 100 | 6-8 |
| 200 | 4-5 |

### Beispiel 5: Zellverträglichkeit von erfindungsgemässen Gewebeklebstoffen

Die Gewebeklebstoffpräparationen aus Beispiel 4 wurden nach Redl et al., Med. Welt 36, 769-776, 1985, auf ihre Zellverträglichkeit überprüft. Als Negativkontrolle diente ein bekannt zytotoxisches Gewebeklebstoffpräparat entsprechend EP-A-0 085 923.

### Ergebnisse:

Sämtliche Präparationen aus Beispiel 4 (mit einem Gehalt an Niacinamid bis zu 200 mmol/l) erwiesen sich als gut zellverträglich. Das Gewebeklebstoffpräparat entsprechend EP-A-0 085 923 führte hingegen erwartungsgemäss zu schwerer Zellschädigung innerhalb weniger Minuten, wodurch die Empfindlichkeit des Testsystems sichergestellt war.

### Beispiel 6:

Ein gereinigtes lyophilisiertes Fibrinogen-Präparat (Bulk-Material) wurde im wesentlichen nach L.A. Kazal et al, Proc. Soc. Exp. Biol. Med. 113, 989-994, 1963, durch Glycinfällung aus einer Fibrinogen enthaltenden Humanplasmafraktion hergestellt.

Dieses Material wurde auf eine Restfeuchte von 7 bis 8 % eingestellt und zur weiteren Virusinaktivierung unter N₂-Atmosphäre 10 h lang auf 60°C, und danach 3 h auf 80°C erhitzt.

Die Analyse des Materials ergab folgende Zusammensetzung:

| | |
|---|---|
| Protein | 73,0% (g/g) |
| Gerinnbares Protein (Fibrinogen) | 69,4% (g/g) |
| Na₃-Citrat.2H₂O | 19,5% (g/g) |

Aus Aliquots dieses Materials wurden Lösungen, enthaltend 22 g Fibrinogen und 10 g Humanalbumin pro Liter, pH 7,4, mit unterschiedlichem Gehalt an Niacinamid hergestellt, wobei die Osmolarität der Lösungen durch Zugabe von NaCl auf jeweils etwa 300 mOsm eingestellt wurde.

Nach Sterilfiltration wurden die Lösungen zu je 50 ml in 125 ml-Flaschen gefüllt und lyophilisiert.

Die so erhaltenen lyophilisierten Fibrinogen-Präparationen wurden mit je 50 ml H₂O bei Raumtemperatur unter leichtem Umschwenken von Hand gelöst und die Rekonstitutionszeiten bestimmt. Dabei wurde der Endpunkt sehr streng definiert: Eine Präparation wurde erst dann als gelöst betrachtet, wenn im durchscheinenden Licht kein ungelöstes Partikel mehr sichtbar war. Die Ergebnisse (Mittelwerte aus drei Bestimmungen) sind in der folgenden Tabelle zusammengestellt:

| **Zusatz** | **Niacinamid- Konzentration mmol/l** | **mmol/g Fibrinogen** | **Rekonstitutionszeit bei RT (Min.)** |
|---|---|---|---|
| 0 (Vergleich) | 0 | 0 | 21 |
| **Niacinamid** | 50 | 2,3 | 13 |
| Niacinamid | 100 | 4,5 | 7 |

Das Beispiel zeigt den günstigen Einfluss erfindungsgemässer löslichkeitverbessernder Substanzen auf die Auflösbarkeit von erhitzten lyophilisiertem Fibrinogen-Präparationen, die entsprechend Infusionspräparaten zusammengesetzt und zur optimalen Verträglichkeit nach Rekonstitution etwa isoton sind.

### Referenzbeispiel:

Eine fibrinogenhältige humane Plasmafraktion wurde mit Tween 80 behandelt, wie bereits im wesentlichen in der AU-B-18306/92 beschrieben, um Viren, wie HIV, zu inaktivieren, die möglicherweise im menschlichen Plasma vorkommen können.

Nach dem ersten Virusinaktivierungsschritt wurde das Material weiters durch eine Präzipitation mit Glycin gereinigt, wie in Beispiel 6 beschrieben. Das Präzipitat wurde mit einer 25 mM Natriumzitratlösung pH 7,3 bei 0-2°C gewaschen. Das gereinigte Präzipitat wurde zu einer Lösung von 50g Protein und 10 mmol Natriumzitrat (pH 7.3) pro Liter aufgelöst und lyophilisiert.

Das lyophilisierte Rohmaterial wurde dann auf eine Feuchtigkeit von 7-8% eingestellt und zur weiteren Virusinaktivierung erhitzt. Das Erhitzen erfolgte unter Stickstoffatmosphäre über eine Dauer von 10 Stunden bei 60°C und anschließend von 1 Std. bei 80°C.

Anschließend wurde das Material mit einer Lösung enthaltend 40 mmol Histidin.HCl, 40 mmol Niacinamid, 80 mg Tween 80 und 100 000 KIE Aprotinin/l zu einer Proteinkonzentration von 40 g/l aufgelöst und der pH mit NaOH auf pH 7,3 eingestellt. Virusinaktiviertes humanes Albumin (Fa. IMMUNO) wurde zugesetzt (6 g/l) sowie humaner gereinigter Faktor XIII, welcher nach der Vorschrift der A 1548/93 zur Virusinaktivierung behandelt wurde (15 000 E/l).

Die Lösung wurde steril filtriert und zu je 2,5 ml in sterile Endbehälter (Glasflaschen) abgefüllt und lyophilisiert. Nach Rekonstitution mit jeweils 1,0 ml Wasser oder Aprotininlösung wurde eine gebrauchsfertige Gewebeklebstofflösung erhalten, die pro ml 90 mg Fibrinogen, 2,5 mg Fibronektin, 15 mg Albumin, 100 µmol Histidin und 100 µmol Niacinamid enthielt. Die durchschnittliche Rekonstitutionszeit bei Raumtemperatur wurde wie in Beispiel 4 beschrieben bestimmt und betrug nur 4 Minuten.

Dieses Beispiel zeigt, daß eine Kombination von löslichkeitsverbessernden Substanzen, die erfindungsgemäß eingesetzt werden, sich besonders eignet zur Herstellung von gut löslichen lyophilisierten Gewebeklebstoffpräparationen, die gleichzeitig ein hohes Maß an Virussicherheit garantieren durch die beschriebenen zwei unterschiedlichen und unabhängigen Maßnahmen zur Inaktivierung von Viren im Zuge des Herstellungsverfahrens.

## Patentansprüche

1. Lagerstabiles Fibrinogenpräparat in lyophilisierter Form oder in flüssig-tiefgefrorener Form zur raschen Bereitung einer gebrauchsfertigen Gewebeklebstofflösung, **dadurch gekennzeichnet, dass** das Präparat eine die Löslichkeit von Fibrinogen verbessernde Substanz enthält und diese Substanz ausgewählt ist aus Nukleinbasen, Nukleoside oder Nukleotide und Benzoesäure, p-Aminobenzoesäure (Vitamin H'), p-Aminosalicylsäure, Hydroxybenzoesäure, Hydroxysalicylsäure, Phenylalanin, Prokain, Niacin, Niacinamid, Picolinsäure, Vitamin B6 (Pyridoxin), Hydroxypyridinen, Pyridin-dicarbonsäuren, Pyridinsulfonsäuren, Piperidin-carbonsäureester, Pyrimidin, Barbitursäure, Uracil, Uridin, Uridinphosphaten, Thymin, Cytosin, Cytidin, Hydroxypyrimidinen, Thiamin (Vitamin B1), Morpholin, Pyrrolidon, Imidazol, Hydantoin, Pyrazol-dicarbonsäuren, Phenazon, Adenosin, Adenosin-Phosphaten, Inosin, Guanosin-Phosphaten, Brenzschleimsäure (Furan-2-carbonsäure), Ascorbinsäure (Vitamin C) und Xantosin."

2. Lagerstabiles Fibrinogenpräparat in lyophilisierter Form gemäß Anspruch 1, **dadurch gekennzeichnet, dass**
(i) die Rekonstitutionszeit bei Auflösen mit Wasser bei Raumtemperatur zu einer Lösung mit einem Fibrinogengehalt von mindestens 70 mg/ml bis zu 15 Minuten, vorzugsweise weniger als 7 Minuten beträgt, und
(ii) die aus dem Präparat erhaltene gebrauchsfertige Gewebeklebstofflösung nach Mischen mit einer Thrombin-CaCl₂-Lösung Fibrinclots mit physiologischer Fibrinstruktur ausbildet.

3. Lagerstabiles Fibrinogenpräparat in flüssigtiefgefrorener Form gemäß Anspruch 1, **dadurch gekennzeichnet, dass**
(i) dieses Präparat bei einer Temperatur von 0 bis 25°C, vorzugsweise unter 20°C, zu einer Lösung mit einem Fibrinogengehalt von mindestens 70 mg/ml verflüssigbar ist, und
(ii) die aus dem Präparat erhaltene gebrauchsfertige Gewebeklebstofflösung nach Mischen mit einer Thrombin-CaCl₂-Lösung Fibrinclots mit physiologischer Fibrinstruktur ausbildet.

4. Präparat nach einem der Ansprüche 1 - 3, **dadurch gekennzeichnet, dass** die genannte Substanz in der gewählten Konzentration im Präparat keine zytotoxische Wirkung hat.

5. Präparat nach einem der Ansprüche 1 - 4, **dadurch gekennzeichnet, dass** die die Löslichkeit von Fibrinogen verbessernde Substanz ausgewählt ist aus der Gruppe der Nukleinbasen, Nukleoside oder Nukleotide.

6. Präparat nach einem der Ansprüche 1-4, **dadurch gekennzeichnet, dass** es sich bei der die Löslichkeit von Fibrinogen verbessernden Substanz um Benzoesäure, p-Aminobenzoesäure (Vitamin H'), p-Aminosalicylsäure, Hydroxybenzoesäure, Hydroxysalicylsäure, Phenylalanin, Prokain, Niacin, Niacinamid, Picolinsäure, Vitamin B6 (Pyridoxin), Hydroxypyridine, Pyridin-dicarbonsäuren, Pyridinsulfonsäuren, Piperidin-carbonsäureester, Pyrimidin, Barbitursäure, Uracil, Uridin, Uridin-Phosphate, Thymin, Cytosin, Cytidin, Hydroxyprimidine, Thiamin (Vitamin B1), Morpholin, Pyrrolidon, Imidazol, Hydantoin, Pyrazol-dicarbonsäuren, Phenazon, Adenosin, Adenosin-Phosphate, Inosin, Guanosin-Phosphate, Brenzschleimsäure (Furan-2-carbonsäure), Ascorbinsäure (Vitamin C) und Xantosin handelt.

7. Präparat nach einem oder mehreren der vorhergehenden Ansprüche, **dadurch gekennzeichnet, dass** die Substanz in einer Menge von 0,03 bis 3 mmol, vorzugsweise von 0,7 bis 1,4 mmol/g Fibrinogen enthalten ist.

8. Präparat nach einem oder mehreren der vorhergehenden Ansprüche, **dadurch gekennzeichnet, dass** die Gewebeklebstofflösung nach dem Mischen mit dem gleichen Volumen einer Thrombin-CaCl₂-Lösung, im wesentlichen bestehend aus 4 I.E. Thrombin und 40 µmol CaCl₂ pro ml, nach längstens 10 Minuten bei 37°C einen undurchsichtigen und zähelastischen Fibrinclot ausbilden kann.

9. Präparat nach einem oder mehreren der vorhergehenden Ansprüche, **dadurch gekennzeichnet, dass** die Gewebeklebstofflösung keine zytotoxische Wirkung hat.

10. Präparat nach einem oder mehreren der vorhergehenden Ansprüche, **dadurch gekennzeichnet, dass** nach Verdünnen der Gewebeklebstofflösung mit dem gleichen Volumen einer isotonen Natriumchloridlösung keine schädigende Wirkung auf Fibroblasten nachweisbar ist

11. Gebrauchsfertige Gewebeklebstofflösung, erhältlich durch Rekonstituieren bzw. Verflüssigen des Präparates nach einem oder mehreren der Ansprüche 1 bis 10.

12. Lyophilisiertes Fibrinogenpräparat, enthaltend eine die Löslichkeit von Fibrinogen verbessernde Substanz, ausgewählt aus der Gruppe der Nukleinbasen, Nukleoside oder Nukleotide oder der folgenden Gruppe:
Benzoesäure, p-Aminobenzoesäure (Vitamin H'), p-Aminosalicylsäure, Hydroxybenzoesäure, Phenylalanin, Procain, Niacin, Niacinamid, Picolinsäure, Vitamin B6 (Pyridoxin), Hydroxypyridinen, Pyridindicarbonsäuren, Pyridinsulfonsäuren, Piperidin-carbonsäureester, Pyrimidin, Barbitursäure, Uracil, Uridin, Uridin-Phosphaten, Thymin, Cytosin, Cytidin, Hydroxypyrimidinen, Thiamin (Vitamin B1), Morpholin, Pyrrolidon, Imidazol, Histidin, Hydantoin, Pyrazol-dicarbonsäuren, Phenazon, Adenosin, Adenosin-Phosphaten, Inosin, Guanosin-Phosphaten, Brenzschleimsäure (Furan-2-carbonsäure), Ascorbinsäure (Vitamin C) und Xantosin.

13. Fibrinogenpräparat nach Anspruch 12, **dadurch gekennzeichnet, dass** die Substanz in einer Menge von 0,12 bis 12 mmol/g Fibrinogen enthalten ist.

14. Fibrinogenpräparat nach Anspruch 12, **dadurch gekennzeichnet, dass** die Substanz in einer Menge von 0,28 bis 5,6 mmol/g Fibrinogen enthalten ist.

## Claims

1. Storage-stable fibrinogen preparation in lyophilised form or in liquid deep-frozen form for rapid preparation of a ready-to-use tissue adhesive solution, **characterised in that** the preparation contains a substance which improves the solubility of fibrinogen and this substance is selected from nucleic bases, nucleosides or nucleotides and benzoic acid, p-aminobenzoic acid (vitamin H'), p-aminosalicylic acid, hydroxybenzoic acid, hydroxysalicylic acid, phenylalanine, procaine, niacin, niacinamide, picolinic acid, vitamin B6 (pyridoxine), hydroxypyridines, pyridine dicarboxylic acids, pyridine sulphonic acids, piperidine carboxylates, pyrimidine, barbituric acid, uracil, uridine, uridine phosphates, thymine, cytosine, cytidine, hydroxypyrimidines, thiamine (vitamin B1), morpholine, pyrrolidone, imidazole, hydantoin, pyrazole dicarboxylic acids, phenazone, adenosine, adenosine phosphates, inosine, guanosine phosphates, pyromucic acid (furan-2-carboxylic acid), ascorbic acid (vitamin C) and xantosine.

2. Storage-stable fibrinogen preparation in lyophilised form according to claim 1, **characterised in that**
(i) the reconstitution time when dissolving with water at room temperature to form a solution having a fibrinogen content of at least 70 mg/ml is up to 15 minutes, preferably less than 7 minutes, and
(ii) the ready-to-use tissue adhesive solution obtained from the preparation, after mixing with a thrombin-CaCl₂ solution, forms fibrin clots with physiological fibrin structure.

3. Storage stable fibrinogen preparation in liquid deep-frozen form according to claim 1, **characterised in that**
(i) this preparation can be liquefied at a temperature of 0 to 25°C, preferably below 20°C, to form a solution having a fibrinogen content of at least 70 mg/ml, and
(ii) the ready-to-use tissue adhesive solution obtained from the preparation, after mixing with a thrombin-CaCl₂ solution, forms fibrin clots with physiological fibrin structure.

4. Preparation according to one of claims 1 - 3, **characterised in that** the said substance has no cytotoxic effect in the preparation at the selected concentration.

5. Preparation according to one of claims 1 - 4, **characterised in that** the substance which improves the solubility of fibrinogen is selected from the group of nucleic bases, nucleosides or nucleotides.

6. Preparation according to one of claims 1-4, **characterised in that** the substance which improves the solubility of fibrinogen is benzoic acid, p-aminobenzoic acid (vitamin H'), p-aminosalicylic acid, hydroxybenzoic acid, hydroxysalicylic acid, phenylalanine, procaine, niacin, niacinamide, picolinic acid, vitamin B6 (pyridoxine), hydroxypyridines, pyridine dicarboxylic acids, pyridine sulphonic acids, piperidine carboxylates, pyrimidine, barbituric acid, uracil, uridine, uridine phosphates, thymine, cytosine, cytidine, hydroxypyrimidines, thiamine (vitamin B1), morpholine, pyrrolidone, imidazole, hydantoin, pyrazole dicarboxylic acids, phenazone, adenosine, adenosine phosphates, inosine, guanosine phosphates, pyromucic acid (furan-2-carboxylic acid), ascorbic acid (vitamin C) and xantosine.

7. Preparation according to one or more of the preceding claims, **characterised in that** the substance is present in a quantity of 0.03 to 3 mmoles, preferably of 0.7 to 1.4 mmoles/g of fibrinogen.

8. Preparation according to one or more of the preceding claims, **characterised in that** the tissue adhesive solution, after mixing with the same volume of a thrombin-CaCl₂ solution, essentially consisting of 4 IU of thrombin and 40 µmoles of CaCl₂ per ml, may form a non-transparent and viscoplastic fibrin clot after 10 minutes at the longest at 37°C.

9. Preparation according to one or more of the preceding claims, **characterised in that** the tissue adhesive solution has no cytotoxic effect.

10. Preparation according to one or more of the preceding claims, **characterised in that** after diluting the tissue adhesive solution with the same volume of an isotonic sodium chloride solution, no damaging effect on fibroblasts can be detected.

11. Ready-to-use tissue adhesive solution which can be obtained by reconstituting or liquefying the preparation according to one or more of claims 1 to 10.

12. Lyophilised fibrinogen preparation containing a substance which improves the solubility of fibrinogen, selected from the group of nucleic bases, nucleosides or nucleotides or the following group:
benzoic acid, p-aminobenzoic acid (vitamin H'), p-aminosalicylic acid, hydroxybenzoic acid, phenylalanine, procaine, niacin, niacinamide, picolinic acid, vitamin B6 (pyridoxine), hydroxypyridines, pyridine dicarboxylic acids, pyridine sulphonic acids, piperidine carboxylates, pyrimidine, barbituric acid, uracil, uridine, uridine phosphates, thymine, cytosine, cytidine, hydroxypyrimidines, thiamine (vitamin B1), morpholine, pyrrolidone, imidazole, hydantoin, pyrazole dicarboxylic acids, phenazone, adenosine, adenosine phosphates, inosine, guanosine phosphates, pyromucic acid (furan-2-carboxylic acid), ascorbic acid (vitamin C) and xantosine.

13. Fibrinogen preparation according to claim 12, **characterised in that** the substance is present in a quantity of 0.12 to 12 mmoles/g of fibrinogen.

14. Fibrinogen preparation according to claim 12, **characterised in that** the substance is present in a quantity of 0.28 to 5.6 mmoles/g of fibrinogen.

## Revendications

1. Préparation de fibrinogène stable au stockage, sous forme lyophilisée ou sous forme surgelée à l'état liquide pour la préparation rapide d'une solution d'adhésif pour tissu, prête à l'utilisation, **caractérisée en ce que** la préparation contient une substance améliorant la solubilité du fibrinogène, et cette substance étant sélectionnée parmi des bases de nucléines, des nucléosides ou des nucléotides et l'acide benzoïque, l'acide p arninobenzoïque (vitamine H'), l'acide p aminosalicylique, l'acide hydroxybenzoïque, l'acide hydroxysalicylique, la phénylalanine, la procaine, la niacine, la niacinamide, l'acide picolinique, la vitamine B6 (pyridoxine), les hydroxypyridines, les acides pyridine dicarbonés, les acides pyridinsulfoniques, les esters d'acide pipéridine carbonés, la pyrimidine, l'acide barbiturique, l'uracile, l'uridine, les phosphates d'uridine, la thymine, la cytosine, la cytidine, les hydroxypyrimidines, la thiamine (vitamine B1), la morpholine, la pyrrolidone, l'imidazole, l'hydantoine, les acides pyrazole dicarbonés, la phénazone, l'adénosine, les phosphates d'adénosine, l'inosine, les phosphates de guanosine, l'acide furoïque (acide furan 2 carboné), l'acide ascorbique (vitamine C) et la xantosine.

2. Préparation de fibrinogène stable au stockage, sous forme lyophilisée, selon la revendication 1, **caractérisée en ce que**
(i) la durée de reconstitution, lors de la dissolution dans l'eau, à la température ambiante, pour donner une solution ayant une teneur en fibrinogène d'au moins 70 mg/ml, est d'une valeur allant jusqu'à 15 minutes, de préférence inférieure à 7 minutes, et
(ii) la solution de colle pour tissu, prête à l'utilisation, obtenue à partir de la préparation, après mélange avec une solution trombine CaCl₂, forme des grumeaux de fibrine, ayant une structure de fibrine physiologique.

3. Préparation de fibrinogène stable au stockage, sous forme surgelée liquide, selon la revendication 1, **caractérisée en ce que**
(i) cette préparation est liquéfiable à une température de 0 à 25°C, de préférence au dessous de 20°C, pour donner une solution ayant une teneur en fibrinogène d'au moins 70 mg/ml, et
(ii) la solution de colle pour tissu, prête à l'utilisation, obtenue à partir de la préparation, après mélange avec une solution trombine CaCl₂, forme des grumeaux de fibrine ayant une structure de fibrine physiologique.

4. Préparation selon l'une des revendications 1 à 3, **caractérisée en ce que** la substance citée n'a aucun effet zytotoxique à la concentration choisie dans la préparation.

5. Préparation selon l'une des revendications 1 à 4, **caractérisée en ce que** la substance, améliorant la solubilité du fibrinogène, est choisie dans le groupe des bases de nucléine, des nucléosides ou nucléotides.

6. Préparation selon l'une des revendications 1 à 4, **caractérisée en ce que,** concernant la substance améliorant la solubilité du fibrinogène, il s'agit d'acide benzoïque, d'acide p aminobenzoïque (vitamine H'), d'acide p amiriosalicylique, d'acide hydroxybenzoïque, d'acide hydroxysalicylique, de phénylalanine, de procaine, de niacine, de niacinamide, d'acide picolinique, de vitamine B6 (pyridoxine), d'hydroxypyridines, d'acides pyridine-dicarbonés, d'acides pyridinsulfoniques, d'esters d'acide pipéridine carbonés, de pyrimidine, d'acide barbiturique, d'uracile, d'uridine, de phosphates d'uridine, de thymine, de cytosine, de cytidine, d'hydroxypyrimidines, de thiamine (vitamine B1), de morpholine, de pyrrolidone, d'imidazole, d'hydantoine, d'acides pyrazole dicarbonés, de phénazone, d'adénosine, de phosphates d'adénosine, d'inosine, de phosphates de guanosine, d'acide furoïque (acide furan 2 carboné), d'acide ascorbique (vitamine C) et de xantosine.

7. Préparation selon l'une ou plusieurs des revendications précédentes, **caractérisée .en ce que** la substance est contenue en une quantité de 0,03 à 3 mmoles, de préférence de 0,7 à 1,4 mmoles/g, de fibrinogène.

8. Préparation selon l'une ou plusieurs des revendications précédentes, **caractérisée en ce que** la solution d'adhésif pour tissu, après le mélange avec le même volume d'une solution trombine CaCl₂, constituée essentiellement de 4 I.E. de trombine et 40 µmoles de CaCl₂ par ml, après une durée maximale de 10 minutes à 37°C, peut former un grumeau de fibrine opaque et ayant une élasticité visqueuse.

9. Préparation selon l'une ou plusieurs des revendications précédentes, **caractérisée en ce que** la solution de colle à tissu n'a aucun effet zytotoxique.

10. Préparation selon l'une ou plusieurs des revendications précédentes, **caractérisée en ce que,** après dilution de la solution d'adhésif pour tissu, avec le même volume d'une solution de chlorure de sodium isotone, aucun effet nuisible sur les fibroblastes ne peut être décelé.

11. Solution d'adhésif pour tissu, prête à l'utilisation, obtenue par reconstitution, respectivement liquéfaction, de la préparation selon l'une ou plusieurs des revendications 1 à 10.

12. Préparation de fibrinogène lyophilisée, contenant une substance améliorant la solubilité du fibrinogène, sélectionnée parmi le groupe des bases de nucléines, des nucléosides ou des nucléotides ou bien du groupe suivant :
l'acide benzoïque, l'acide p aminobenzoïque (vitamine H'), l'acide p aminosalicylique, l'acide hydroxybenzoïque, la phénylalanine, la procaïne, la niacine, la niacinamide, l'acide picolinique, la vitamine B6 (pyridoxine), les hydroxypyridines, les acides pyridine dicarbonés, les acides pyridinsulfoniques, les esters d'acide pipéridine carbonés, la pyrimidine, l'acide barbiturique, l'uracile, l'uridine, les phosphates d'uridine, la thymine, la cytosine, la cytidine, les hydroxypyrimidines, la thiamine (vitamine B1), la morpholine, la pyrrolidone, l'irnidazole, l'hydantoine, les acides pyrazole dicarbonés, la phénazone, l'adénosine, les phosphates d'adénosine, l'inosine, les phosphates de guanosine, l'acide furoïque (acide furan 2 carboné), l'acide ascorbique (vitamine C) et la xantosine.

13. Préparation de fibrinogène selon la revendication 12, **caractérisée en ce que** la substance est contenue en une quantité de 0,12 à 12 mmoles/g de fibrinogène.

14. Préparation de fibrinogène selon la revendication 12, **caractérisée en ce que** la substance est contenue en une quantité de 0,28 à 5,6 mmoles/g de fibrinogène.
